Europäisches Patentamt

European Patent Office

Office européen des brevets

(11) Veröffentlichungsnummer: **0 018 550**
**B1**

(12) **EUROPÄISCHE PATENTSCHRIFT**

(45) Veröffentlichungstag der Patentschrift: 09.10.85

(21) Anmeldenummer: 80102069.4

(22) Anmeldetag: 17.04.80

(51) Int. Cl.⁴: **A 61 K 9/00**, A 61 K 9/48, A 61 K 31/14, A 61 K 31/195

(54) Mittel zur Behandlung von Muskelerkrankungen.

(30) Priorität: 17.04.79 DE 2915433

(43) Veröffentlichungstag der Anmeldung:
12.11.80 Patentblatt 80/23

(45) Bekanntmachung des Hinweises auf die Patenterteilung:
09.10.85 Patentblatt 85/41

(84) Benannte Vertragsstaaten:
DE

(56) Entgegenhaltungen:
EP-A-0 001 924
DE-C- 593 271
DE-C- 831 879
FR-A-2 183 012
US-A-2 556 688
US-A-3 819 480

Die Akte enthält technische Angaben, die nach dem Eingang der Anmeldung eingereicht wurden und die nicht in dieser Patentschrift enthalten sind.

(73) Patentinhaber: PLANTORGAN WERK Heinrich G.E. Christensen KG
Hornbusch 1
D-2903 Bad Zwischenahn (DE)

(72) Erfinder: Hort, Walter, Dr.med.
Kaiserstrasse 7
D-6600 Saarbrücken (DE)

(74) Vertreter: Kinzebach, Werner, Dr. et al
Patentanwälte Reitstötter, Kinzebach und Partner Postfach 780
D-8000 München 43 (DE)

Courier Press, Leamington Spa, England.

**Beschreibung**

Die Ursache der Muskelverletzungen, ob es sich um eine sogenannte Muskelzerrung oder einen Muskelfaseriß oder um eine Muskelhärte im Sinne von Myogelose oder Myalgie handelt, wird im allgemeinen auf eine Koordinationsstörung, örtliche Übermüdung, mangelhafte Durchblutung, sei es durch Kälte, ungenügendes Training, Übermüdung nach dem Training, unzureichende Vorbereitung vor Beginn der sportlichen Betätigung oder sogar auf Infektionsherde und frühere Infektionskrankheiten zurückgeführt.

Die eigentliche Pathogenese kann sicherlich nicht nur in einer mechanischen Überlastung bzw. Überdehnung der Muskelfaser gesehen werden, auch wenn beim Muskelfaseriß eine Kontinuitätsunterbrechung durch vermutliche Überdehnung auftritt. Für die sogenannte Muskelzerrung, die bisher weder elektronenmikroskopisch noch in situ gesehen werden konnte, befriedigt diese Aussage jedoch nicht.

Die bisherige Therapie der Muskelerkrankungen, insbesondere der Muskelverletzungen beruht einzig und allein auf äußeren Maßnahmen, wie Anlegen von Druck- bzw. Stützverbänden, Gabe von Myotonolytika, Wärmeanwendung, elektro-therapeutische Maßnahmen und schließlich Massagen. Diese Therapie wurde auch schon vor 20 Jahren angewandt. Es ist allgemein bekannt, daß die Ergebnisse unbefriedigend sind.

Die Verwendung von Lösungen von komplexen Gemischen von Aminosäuren zur parenteralen Ernährung in Verbindung mit anorganischen Salzen, wie Natrium-, Kalium-, Calcium- und Magnesium- verbindungen in Gegenwart anderer ernährungsphysiologisch wichtiger Substanzen ist bekannt. Entsprechende Lösungen sind in der Roten Liste 1977/78 beschrieben. Dort finden sich unter den Nummern 51001 ff. zahllose Formulierungen komplizierter Gemische für Infusions- und Standardinjektionslösungen, die zur parenteralen Ernährung bzw. Supplementierung bestimmt sind. Die komplexen Aminosäurezusammensetzungen des Standes der Technik sind stets unter ernährungsphysiologischen Gesichtspunkten formuliert. Der Stand der Technik beschrreibt keine Injektionslösungen mit nur einer Aminosäure oder einer Kombination von zwei bis drei Aminosäuren, da derartige Formulierungen zur Lösung der bekannten Aufgabe nicht geeignet sind.

Die US—PS 2 556 688 beschreibt ein Mittel, das aus etwa gleichen Teilen Methionin und einem Salz davon besteht. Diese Zusammensetzung dient dazu, die Konzentration an Methionin in wäßriger Lösung zu erhöhen. Eine Indikation ist nicht angegeben.

Die US—PS 3 819 480 beschreibt ein therapeutisches Mittel, das Methionin und 2-Dimethylaminoäthanol in wäßrigem Medium, enthält. Dieses Mittel erhöht die Lebenserwartung bei Tieren und die in vitro- Überlebenszeit von tierischen Zellen, Geweben und Organen.

Die DE—PS 831 879 beschreibt ein Verfahren zur Überführung von hygroskopischen Salzen des Cholins und seiner Verbindungen in für perorale Darreichung geeignete Form. Die Cholinsalze besitzen parasymphathicuserregende Wirkung.

Es gibt also keinen Stand der Technik, in dem vorgeschlagen oder angeregt wird, Muskelerkrankungen, wie Muskelverletzungen und Muskelhärte durch Verabreichung von Methyldonatoren, insbesondere von Aminosäuren, wie Methionin, zu behandeln.

Der Erfindung liegt die Aufgabe zugrunde, ein wirksames Arzneimittel zur Behandlung von Muskelerkrankungen, insbesondere von Muskelverletzungen und Muskelhärte, zu schaffen.

Diese Aufgabe wird gelöst durch ein Mittel, das dadurch gekennzeichnet ist, daß es als Wirkstoff ein Gemisch aus Methionin und Cholin zusammen mit üblichen pharmazeutisch verträglichen Verdünnungsmitteln, Trägern und/oder Adjuvantien enthält.

Nach einer bevorzugten Ausführungsform liegt das erfindungsgemäße Mittel vor

(a) in Form einer wäßrigen, injizierbaren Lösung, oder

(b) in einem pharmazeutisch verträglichen Träger oder Bindemittel zur oralen Verabreichung.

Es ist zweckmäßig, Methionin und Cholin zur Zubereitung einer wäßrigen, injizierbaren Lösung in einer Menge von 5 bis 80 mg/ml Lösung, beispielsweise 20 bis 60 mg/ml Lösung zu verwenden.

Besonders günstig ist die Formulierung des erfindungsgemäßen Mittels in Form oral verabreichbarer Kapseln, da bei dieser Art der Formulierung keinerlei Geschmacksbeeinträchtigungen bei der Einnahme auftreten können.

Zweckmäßig ist auch ein Mittel, das Methionin und Cholin in einer Menge von 2 bis 8 g auf 250 ml Lösung zur Herstellung einer Einheitsdosis enthält.

Als Zusatz zu den aktiven Wirkstoffen der erfindungsgemäßen Mittel kommen auch die anderen Einzelsubstanzen Histidin, Isoleucin, Leucin, Lysinacetat und Phenylalanin in Betracht.

Man kann DL-Methionin verwenden, die aufgetrennten Isomeren sind jedoch als solche ebenfalls brauchbar.

Die Erfindung geht von der neuen Erkenntnis aus, daß man vom rein mechanischen Denken bei den Muskelerkrankungen, insbesondere bei den Muskelverletzungen abgehen muß. Die physiologische Funktion der Muskeln besteht darin, durch Kontraktion mechanische Arbeit zu leisten. Die Kontraktilität der Muskelfaser setzt zwei Einrichtungen voraus: Strukturelemente, die sich reversibel verkürzen können und einen chemischen Mechanismus, durch den die Verkürzung bewirkt und wieder rückgängig gemacht wird.

Das Element jeder Muskelfunktion ist also die einfache Zuckung der einzelnen Faser: eine Verkuŕzung mit nachfolgender Erschlaffung (sogenannter Arbeitszyklus der Muskelfaser).

Energieträger des Kohlehydratstoffwechsels des Muskels ist das Muskelglycogen. Daneben spielen für die Kontraktion der Muskelfaser das Adenosin-Triphosphat (ATP) und das Phosphat-Kreatin die entscheidende Rolle, denn die einzige Form chemischer Energie, die vom kontraktilen Protein in mechanische Arbeit umgewandelt werden kann, ist die Energie, die bei der hydrolytischen Abspaltung der Phosphatgruppe ATP frei wird. Die ATP-Reserven der Muskeln nehmen selbst bei schwerer Arbeit nicht meßbar ab, denn das entstehende Adenosin-Diphosphat (ADP) wird sehr schnell aus dem Phospho-Kreatin-Vorrat rephosphoryliert. Der Vorrat an Phospho-Kreatin ist begrenzt, da das Kreatin nur im Blutkreislauf vorhanden ist, wenn die Methylgruppe eines Methyldonators, beispielsweise von Methionin, zur Methylierung der Guanidin-Essigsäure vorhanden ist.

Laut F. Leuthart, Lehrbuch der physiol. Chemie, Walter de Gruyter & Co., 1961, ist auch das ATP bei den Trans-Methylierungsreaktionen des Methionins im Spiel (vgl. Figur 1).

Die Wirkungen des ATP neben der Kontraktionseigenschaft sind folgende:

Es beeinflußt die elastischen Eigenschaften des Muskels in dem Sinne, daß es ihn weicher und dehnbarer macht, d.h. ohne ATP ist der Muskel starr und wenig dehnbar. ATP hat also eine sogenannte Weichmacherwirkung auf die Muskulatur. Das aus dem ATP entstehende ADP hat jedoch eine tonisierende und erstarrende Wirkung auf die Muskulatur.

Nach der sogenannten Lohmann-Reaktion: Phospho-Kreatin plus ADP = Kreatin plus ATP kann man folgern, daß bei fehlendem Phospho-Kreatin und demnach nicht erfolgter Methylierung der Guanidin-Essigsäure ein Mangel an ATP auftreten kann.

In unseren Breitengraden ist eine mangelhafte Versorgung der Muskulatur mit Nahrungsstoffen normalerweise jedoch nicht gegeben und nicht Ursache für eine unzureichende ATP-Synthese. Häufig dagegen wird die ATP-Synthese durch Sauerstoffmangel als Folge unzureichender Durchblutung, wie Gefäßverengung, Gefäßverschlüsse, aber auch durch starke Muskelkontraktion während harter Arbeit eingeschränkt.

Aus dem oben Gesagten kann man folgern, daß der ATP- und der Phospho-Kreatin-Spiegel in Ruhe normal sind. Bei stärkerer Belastung fällt jedoch bei nahezu konstantem ATP-Spiegel der Phospho-Kreatin-Spiegel deutlich ab.

Erfindungsgemäß werden Arzneimittelformulierungen geschaffen, die zur Injektion, insbesondere zur parenteralen Verabreichung und auch zur oralen Verabreichung besonders geeignet sind. Die parenterale Verabreichung erfolgt zweckmäßig bei akuten Muskelverletzungen. Gute Erfolge werden erzielt, wenn am Tag der Erstbehandlung 250 ml Lösung parenteral verabreicht werden, gefolgt von parenteraler Verabreichung derselben Menge am darauffolgenden Tag. Falls dann noch Beschwerden bestehen, kann eine dritte Infusion in derselben Höhe nach einer Woche verabreicht werden. Bei sofortiger Besserung der Beschwerden reichen selbstverständlich eine oder zwei Infusionen aus.

Die Infusionslösungen könen beispielsweise 1,5 bis 24 g aktiven Bestandteil pro 250 ml Infusionslösung enthalten. Wenn an drei aufeinanderfolgenden Tagen eine Verabreichung durch Injektion erfolgt, so sollten pro Tag etwa 2 g aktiver Wirkstoff zugeführt werden.

Zur oralen Verabreichung empfiehlt sich eine Formulierung in geschmacksneutraler Kapselform, beispielsweise in einer Kapsel aus Hartgelatine. Orale Formulierungen können auch in Form eines Sirups vorliegen. Der Sirup kann Geschmacksstoffe, insbesondere Glucose, und Verdickungsmittel, sowie andere übliche Zusätze enthalten.

Bisweilen erweist es sich als zweckmäßig, noch andere Zusätze, beispielsweise Ascorbinsäure, Vitamin D 2 oder Vitamin B 6, sowie üliche Zusätze, wie Metallcitrate und dergleichen, mit zu verwenden.

Geeignete tägliche Dosismengen liegen im Bereich von etwa 0,5 bis 16 g aktive Substanz, je nach Lage des einzelnen Falles.

Die erfindungsgemäßen Mittel erweisen sich als wirksam bei der Behandlung von Muskelerkrankungen im allgemeinen d. h. bei der Behandlung aller von der Muskulatur ausgehender Beschwerdebilder, z. B. bei Muskelverletzungen und Muskelhärte, jedoch auch zur gegebenenfalls prophylaktischen Behandlung von Muskelrheumatismus oder von Muskelbeschwerden bei Schilddrüsenerkrankungen.

Bisher wurde bei 60 Patienten eine Infusionstherapie unter Anwendung eines erfindungsgemäß erhältlichen Arzneimittels mit Methionin/Cholin als aktivem Wirkstoff durchgeführt. Ebenso kamen parenterale Arzneimittelformulierungen zur Anwendung. Im Falle akuter Verletzungen wurde hauptsächlich mit parenteralen Lösungen therapiert. Am tage der Erstbehandlung wurden 250 ml Lösung eingesetzt, am darauffolgenden Tag dieselbe Menge, sowie, falls erforderlich, nach einer Woche nochmals dieselbe Dosis. Bei sofortiger Besserung der Beschwerden wurden nur eine oder zwei Infusionen verabreicht.

Es Handelte sich bei den Patienten um solche mit frischen Zerrungeh und frischen Muskelfaserrissen, bei denen diese Diagnose klinisch gestellt war. Ebenfalls behandelt wurden Patienten mit alten Muskelfaserrissen bzw. sogenannten Muskelzerrungen und Patienten mit isolierten Myogelosen bzw. Verhärtungen der Muskulatur. Die hierbei erzielten Behandlungsergebnisse sind in der nachstehenden Tabelle zusammengefaßt:

## TABELLE

| Art der Muskel-verletzungen | Anzahl Patienten | Ende der Ruhe-schmerzen (Tage) | Ende der Geh-schmerzen (Tage) | Beginn der Sportfähig-keit (Tage) | Vergleichs-gruppe ohne Methionin/Cholin (klassische Behandlung) * Ende der Ruhe-schmerzen (Tage) |
|---|---|---|---|---|---|
| Muskelfaserrisse | 20 | 2,3 | 7 | 25 | 6 bis 8,2 |
| Muskelzerrungen | 22 | 1,8 | 4,5 | 10 | 3 bis 4 |
| ältere Ver-letzungen Verhärtungen | 18 | 0 | 0 | 4 | monatelange Schmerzen |

*Angaben der Fachliteratur entnommen.

Bei 20 Patienten mit Muskelfaserrissen wurde durch die vorstehende Behandlung bereits nach einem Mittel von 2,3 Tagen Schmerzfreiheit in Ruhe erzielt. Im Vergleich hierzu bedurfte es bei klassischer Behandlung einer Vergleichsgruppe von Patienten eines längeren Zeitraums, nämlich 6 bis 8,2 Tage. Diie mit dem erfindungsgemäßen Arzneimittel behandelten Patienten hatten nach knapp 7 Tagen keine Gehschmerzen mehr, ab 25 Tagen waren sie wieder sportfähig (Laufen etc.).

Bei 22 Patienten mit sogenannten Muskelzerrungen wurde unter Anwendung des erfindungsgemäßen Mittels Schmerzfreiheit in Ruhe bereits nach durchnittlich 1,8 Tagen erzielt. Das Ende der Gehschmerzen trat im Mittel nach 4,5 Tagen ein, beim Laufen bestand Schmerzfreiheit nach etwa 10 Tagen. Im Vergleich hierzu wurden bei einer Vergleichsgruppe, die auf klassische Weise behandelt wurde, Schmerzfreiheit in Ruhe erst nach 3 bis 4 tagen festgestellt.

Ähnlich hervorragende Ergebnisse konnten erfindungsgemäß auch bei Muskelverhärtungen und bei alten Rissen bzw. sogenannten zerrungen erzielt werden.

Die nachfolgenden Beispiele dienen zur weiteren Erläuterung der Erfindung.

### Beispiel 1

Parenterale Infusionslösung:

| | |
|---|---|
| Cholin | 2,5 g |
| Methionin | 2,5 g |
| steriles Wasser auf | 200,0 g |

### Beispiel 2

Orales Mittel in Kapselform:

| | |
|---|---|
| Methionin | 2,5 g |
| Cholin | 2,5 g |

konfektioniert in Hartgelatinekapseln.

**Patentansprüche**

1. Mittel zur Behandlung von Muskelerkrankungen, dadurch gekennzeichnet, daß es als Wirkstoff ein Gemisch aus Methionin und Cholin zusammen mit üblichen pharmazeutisch verträglichen Verdünnungsmitteln, Trägern und/oder Adjuvantien ennthält.

2. Mittel nach Anspruch 1 in Form einer wäßri-gen, injizierbaren Lösung, dadurch gekennzeich-net, daß es Methionin und Cholin in einer Menge von 5 bis 80 mg/ml Lösung enthält.

3. Mittel nach einem der Ansprüche 1 oder 2 in Form einer Einheitsdosis, dadurch gekennzeichnet, daß es 2 bis 8 g Wirkstoff in 250 ml Lösung enthält.

4. Mittel nach Anspruch 1 in Form von oral verabreichbaren Kapseln.

5. Verfahren zur Herstellung eines Mittels nach einem der Ansprüche 1 bis 4, dadurch gekennzeichnet, daß man Methionin und Cholin mit pharmazeutisch verträglichen Verdünnungsmitteln, Trägern und/oder Adjuvantien vermischt.

**Revendications**

1. Composition pour le traitement de maladies musculaires, charactériseé par le fait, qu'elle contient, à titre de principe actif, un mélange de methionine et de choline ensemble avec de diluants, véhicules et/or adjuvants pharmaceutiquement acceptables.

2. Composition selon la revendication 1 en forme d'une solution aqueuse et injectable charactériseé par le fait qu'elle contient de la méthionine et de la choline à raison de 5 à 80 mg/ml solution.

3. Composition selon l'une des revendications 1 ou 2 en forme d'une dose unique, characterisée qu'elle contient 2 à 8 g de principe actif dans 250 ml de solution.

4. Composition selon la revendication 1 en-forme de capsules pour l'administration orale.

5. Procédé pour la préparation d'une composition selon une des revendications 1 à 4 characté-risé par le fait, que l'un mélange de la méthionine et de la choline avec des diluants, véhicules et/or adjuvants pharmaceutiquement acceptables.

**Claims**

1. Composition for treating muscular diseases, characterized in that it contains as active ingredient a mixture out of methionine and choline together with common pharmaceutically acceptable diluents, carriers and/or adjuvants.

2. Composition according to claim 1 in the form of an aqueous, injectable solution, characterized in that it contains methionine and cholin in an amount of 5 to 80 mg/ml.

3. Composition according to one of the claims 1 or 2 in the form of a unit dosage, characterized in that it contains 2 to 8 g active ingredient in 250 ml solution.

4. Composition according to claim 1 in the form of capsules to be orally administered.

5. Process for producing a composition according to one of the claims 1 to 4, characterized in that one mixes methionine and choline together with pharmaceutically acceptable diluents, carriers and/or or adjuvants.